# EUROPÄISCHE PATENTSCHRIFT

(11) **EP 0 646 372 B1**
(45) Veröffentlichungstag und Bekanntmachung des Hinweises auf die Patenterteilung: **31.03.1999**
(21) Anmeldenummer: 94810524.2
(22) Anmeldetag: 09.09.1994
(51) Int. Cl.: A61K 31/165, A61K 9/00

(54) **Capsaicin enthaltendes Arzneimittel zur Behandlung chronischer Rhinopathie**
Medicine containing capsaicin for the treatment of chronic rhinopathy
Médicament à base de capsaicine pour le traitement de la rhinopathie chronique

(30) Priorität: 30.09.1993 CH 2940/93
(43) Veröffentlichungstag der Anmeldung: 05.04.1995
(73) Patentinhaber: MEDICHEMIE AG, CH-4107 Ettingen/Basel (CH)
(72) Erfinder: Widauer, Josef Olaf, CH-4123 Allschwil (CH)
(74) Vertreter: Hug Interlizenz AG

(56) Entgegenhaltungen:
- EP-A- 0 401 903
- US-A- 5 008 289

## Beschreibung

### TECHNISCHES GEBIET

Die vorliegende Erfindung betrifft ein flüssiges Arzneimittel enthaltend Capsaicin in Lösung als Wirkstoff zur Behandlung chronischer Rhinopathie.

Capsaicin ist der scharfe Bestandteil der roten Pfefferschote und anderer Nachtschattengewächse. Seine chemische Bezeichung lautet 8-Methyl-N-Vanillyl-6-Nonenamid. Günstige therapeutische Wirkungen dieses Wirkstoffes sind für die unterschiedlichsten Krankheitsbilder bekannt, darunter die vasomotorische bzw. hyperreflektorische Rhinopathie, Cluster-Kopfschmerzen, das Post-Mastoidektomie-Schmerzsyndrom, Magenschleimhautläsionen, die hyperreaktive Zystitis oder die lokale Reaktion der Haut auf oberflachliche Verletzungen. In den USA wurde Capsaicin kürzlich zur topischen Applikation bei postherpetischer Neuralgie und zur Linderung von Schmerzen bei diabetischer Neurophatie, rheumatoider Arthritis und Östeoarthritis zugelassen.

Die hyperreflektorische Rhinopathie ist weder eine infektiöse noch allergische unspezifische Schwellung der Nasenschleimhaut. Neurorezeptorisch vermittelte Irritationen mechanischer (Septumsspron, Septumsdeviation u.a. anatomisch bedingte endonasale Engstellen), thermischer (Kalt-Warm-Wechsel, trockende Raumluft) oder chemischer (Tabakrauch und andere Inhalations-noxen) Art führen über der willkürlichen Steuerung entzogene Reflexmechanismen (Axonreflex) zur behinderten Nasenatmung, Rhinorrhoe, Niesattacken und gelegentlich rhinogenen Kopfschmerzen. Verantwortlich für diese Beschwerden ist eine Störung des Gleichgewichts der autonomen Innervation der Nasenschleimhaut, die zu lokaler Vasodilatation mit Plasmaextravasation, Hypersekretion und Kontraktion glatter Muskelfasern führt. Dabei spielen nicht ausschliesslich eine Überreaktivität des Parasympathikus gegenüber dem Sympathikus, sondern vielmehr neuentdeckte Transmittersubstanzen, u.a. die Neuropeptide Substanz P (SP) und Calcitoin gene related peptide (CGRP) eine wichtige pathogenetische Rolle. Capsaicin führt nach wiederholter lokaler Applikation zu einer spezifischen Desensibilisierung der am oben genannten Axonreflex beteiligten Mechano-, Chemo- und Thermorezeptoren. Neben der Neuropeptidverarmung kommt es dabei zu einer spezifischen Degeneration der SP- und CGRP-reaktiven C-Nervenfasern. Es resultiert eine Reduktion der hyperreflektorischen Rhinopathiebeschwerden mit Langzeitwirkung. Interessant ist auch die Tatsache, dass in der Nasenschleimhaut echte Capsaicin-Rezeptoren nachgewiesen werden können.

### STAND DER TECHNIK

Bisher war es üblich, das Capsaicin zur Rhinopathiebehandlung in hochkonzentrierter Form zu applizieren. Wegen des durch den Wirkstoff verursachten starken Brennens war dies nur nach vorgängiger Lokalanästhesie der Nasenschleimhaut möglich und musste durch den Arzt ausgeführt werden. Dieser Vorgang musste zudem in unterschiedlichen zeitlichen Abständen mehrmals wiederholt werden. Das Capsaicin wurde dabei in äthanol-, paraffinöl- oder Tween 80-haltigen Lösungen verwendet. Im Lösungsmittel enthaltener Alkohol beeinflusst jedoch die Nasenschleimhaut in Form von direkter chemischer Irritation, Austrocknung durch Wasserentzug, Ausschwemmung von Lipiden sowie durch Störung des Gel-Sol-Verhältnisses des Nasenschleims und des damit verbundenen mukoziliaren Transportes.

Paraffinöl beeinflusst den mukoziliaren Transport durch Hemmung der Zilienfunktion. Tween 80 ist lokal reizend. Bei Verwendung von Lidocain oder Pantocain als Lokalanästhesie bleibt die Nasenatmung unbeeinflusst, Cocain hingegen führt zu einer lokalen Vasokonstriktion. Die gängige Capsaicinbehandlung ist demnach für den Arzt und den Patienten aufwendig und für letzteren mit z.T. sehr unangenehmen Nebenwirkungen und Begleiterscheinungen verbunden.

### DARSTELLUNG DER ERFINDUNG

Im Hinblick auf die vorstehend erwähnten Nachteile der gängigen Capsaicinbehandlung stellt sich die vorliegende Erfindung die Aufgabe, den Wirkstoff in einer Anwendungsform zur Verfügung zu stellen, die ohne Lokalanästhesie und durch den Patienten selbst auf der Nasenschleimhaut lokal applizierbar ist. Diese Aufgabe wird erfindungsgemäss gelöst durch ein flüssiges Arzneimittel gemäss Patentanspruch 1. Das erfindungsgemässe Arzneimittel enthält demnach den Wirkstoff in neutraler, wässriger Lösung sowie in einer vergleichsweise sehr geringen Konzentration.

Vorzugsweise ist das erfindungsgemässe Arzneimittel als Nasenspray, insbesondere als Dosierspray zubereitet. Im letzteren Fall ist bevorzugt, dass pro Spray-Applikation (bzw. Hub bei einem mechanisch betätigten Pumpspray) 0,1 bis 0,5 µg Wirkstoff freigesetzt bzw. zerstäubt werden. In dem genannten Bereich noch weiter bevorzugt sind 0,125 µg und 0,25 µg pro Spray-Applikation (oder Hub).

Nachstehend aufgeführt ist das Ergebnis einer Untersuchung mit erfindungsgemäss zubereiteten Dosierspray an insgesamt 84 Patienten mit klinischen Beschwerden einer hyperreflektorischen Rhinopathie. Die Hauptbeschwerden der Patienten umfassten ganzjährig behinderte Nasenatmung, chronische Rhinorrhoe und Niesattacken. Den Patienten wurde ein 0,125 µg pro Hub freisetzendes Dosierspray nach Hause mitgegeben mit der Aufforderung, während vier Wochen dreimal täglich je zwei Hübe in jedes Nasenloch zu sprayen. Bei einer nach zwei Wochen durchgeführten klinischen Kontrolle wurde bei 10% der Patienten die Applikationsdosis pro Hub auf 0,25 µg erhöht.
- Eine massive Nasenatembehinderung bestand vor der Behandlung bei 64% aller Patienten, danach nur noch bei 9%.
- An massiver oder intermittierender Rhinorrhoe litten vor der Behandlung 58% der Patienten, danach noch 12%. Die diesbezügliche Beschwerdefreiheit konnte von 6 auf 50% erhöht werden.
- 64% der Patienten waren nach der Behandlung frei von Niesanfällen.
- Der Nasenflow (Summe des in- und expiratorischen Volumenstroms in ccm/s bei 150 Pa (I+E 150)) erhöhte sich von 1164 ccm/s vor der Behandlung auf 1393 ccm/s danach.
- Das Seitenverhältnis des Nasenflows (durchschnittliches Seitenverhältnis der besseren zur schlechteren Nasenseite (dSQ)) verminderte sich von 2,11 auf 1,79 nach der Behandlung.

Das Überraschende an den vorstehenden Therapieergebnissen ist die ausgezeichnete Wirksamkeit des Wirkstoffes Capsaicin auch noch bei der sehr niedrigen, hier angewendeten Dosierung. Bei den bisher üblichen Anwendungen lag diese um einen Faktor 40 - 50 höher.

Das Ausreichen der niedrigen Dosierung für den gewünschten therapeutischen Effekt macht den Weg frei, den Wirkstoff in neutraler Lösung zu formulieren. Capsaicin ist nämlich in Wasser sehr schlecht löslich. Zur Aufbereitung von Capsaicin wurden deshalb bisher wohl auch die mit starken Nebenwirkungen behafteten organischen Lösungsmittel verwendet. Unter Verwendung von Polyethylenglykol als Lösungsvermittler lässt sich Capsaicin in Wasser jedoch ohne weiteres bis zu einer Konzentration von etwa 14 µg/ml lösen. Der Polyethylenglykolanteil im fertigen Nasenspray lässt sich dabei unter 1% und damit auf einem Wert halten, der bei den Patienten keine erkennbare Nebenwirkung verursacht.

Durch die geringe Dosierung des Wirkstoffes und das neutrale wässrige Medium, in dem dieser gelöst ist, ergeben sich vor alle folgende Vorteile:
- Das potentiell durch den Wirkstoff auf der Nasenschleimhaut verursachte Brennen tritt bei Anwendung des erfindungsgemässen Nasensprays über einen längeren Zeitraum lediglich in den ersten Tagen und auch dann nur verhältnismässig schwach auf.
- Zur Applikation ist keine vorgängige Lokalanästhesie erforderlich; der Wirkstoff kann durch den Patienten selbst appliziert werden. Die bisher allein dazu erforderlichen Arztbesuche entfallen.

Formulierungsbeispiel für 0,125 µg pro Hub:

Zur Herstellung von insgesamt 1000 ml Nasenspray wird 28,56 mg Capsaicin in 20 g Polyethylenglykol 200 bei erhöhter Temperatur (ca. 50 - 60°C) gelöst. 1,25 g der so gewonnenen Lösung wird zu 1000 ml Wasser verdünnt.

## Patentansprüche

1. Flüssiges Arzneimittel enthaltend Capsaicin in Lösung als Wirkstoff zur Behandlung chronischer Rhinopathie, dadurch gekennzeichnet, dass die Wirkstofflösung eine neutrale, wässrige Lösung mit einer Wirkstoffkonzentration zwischen 0,7 und 14 µg/ml ist.

2. Arzneimittel nach Anspruch 1, dadurch gekennzeichnet, dass es als Nasenspray zubereitet ist.

3. Arzneimittel nach Anspruch 2, dadurch gekennzeichnet, dass es als Nasen-Dosierspray zubereitet ist und dass pro Spray-Stoss 0,1 bis 0,5 µg Wirkstoff freigesetzt bzw. zerstäubt werden.

## Claims

1. Liquid medicine containing capsaicin in solution as the active ingredient for the treatment of chronic rhinopathy, characterized in that the active ingredient solution is a neutral, aqueous solution with an active ingredient concentration between 0.7 and 14 µg/ml.

2. Medicine according to claim 1, characterized in that it is formulated as a nose spray.

3. Medicine according to claim 2, characterized in that it is formulated as a nasal dosing spray and that per spray stroke 0.1 to 0.5 µg of active ingredient is released or atomized.

## Revendications

1. Médicament liquide, contenant de la capsaïcine en solution comme agent actif, pour le traitement de rhinopathie chronique, caractérisé en ce que la solution d'agent actif est une solution aqueuse neutre ayant une concentration en agent actif entre 0,7 et 14 µg/ml.

2. Médicament suivant la revendication 1, caractérisé en ce qu'il est préparé sous forme de spray nasal.

3. Médicament suivant la revendication 2, caractérisé en ce qu'il est préparé sous forme de spray doseur nasal et en ce que 0,1 à 0,5 µg d'agent actif est libéré ou selon le cas pulvérisé à chaque coup de pulvérisation.
